(19) Europäisches Patentamt

European Patent Office

Office européen des brevets

(11) EP 0 514 527 B1

## (12) FASCICULE DE BREVET EUROPEEN

(45) Date de publication et mention
de la délivrance du brevet:
**13.03.1996 Bulletin 1996/11**

(21) Numéro de dépôt: **92901704.4**

(22) Date de dépôt: **10.12.1991**

(51) Int. Cl.$^6$: **B01J 23/62**, C10G 35/09

(86) Numéro de dépôt international: **PCT/FR91/00992**

(87) Numéro de publication internationale:
**WO 92/10289 (25.06.1992 Gazette 1992/14)**

(54) **CATALYSEUR A BASE D'ALUMINE ETA CHLOREE ET SON UTILISATION EN ISOMERISATION DES N-PARAFFINES EN C4-C6**

KATALYSATOR AUF BASIS VON CHLORIERTEM ETA-ALUMINIUMOXID UND DESSEN VERWENDUNG ZUR ISOMERISIERUNG VON C4-C6 N-PARAFFINEN

CHLORINATED ETA ALUMINA-BASED CATALYST AND ITS USE IN ISOMERISATION OF C4-C6 N-PARAFFINS

(84) Etats contractants désignés:
**DE ES GB IT NL**

(30) Priorité: **10.12.1990 FR 9015546**

(43) Date de publication de la demande:
**25.11.1992 Bulletin 1992/48**

(73) Titulaire: **INSTITUT FRANCAIS DU PETROLE**
**F-92502 Rueil-Malmaison (FR)**

(72) Inventeurs:
• **TRAVERS, Christine**
  **F-92500 Rueil-Malmaison (FR)**
• **MARTINO, Germain**
  **F-78300 Poissy (FR)**

(74) Mandataire: **Andreeff, François et al**
**INSTITUT FRANCAIS DU PETROLE**
**4, avenue de Bois-Préau**
**F-92506 Rueil-Malmaison Cédex (FR)**

(56) Documents cités:
**DE-B- 1 276 611          FR-A- 2 137 320**
**FR-A- 2 545 380          US-A- 3 919 340**
**US-A- 4 804 803          US-A- 4 964 975**

Remarques:
Le dossier contient des informations techniques présentées postérieurement au dépôt de la demande et ne figurant pas dans le présent fascicule.

## Description

La présente invention concerne un nouveau catalyseur bimétallique à base d'alumine chlorée et sa mise en oeuvre dans un procédé d'isomérisation de paraffines normales $C_4$ - $C_6$ ne nécessitant pas de recyclage d'hydrogène.

De par la suppression, notamment à des fins écologiques, des alkyls de plomb dans les essences, l'isomérisation des paraffines normales de 4 à 6 atomes de carbone revêt actuellement une importance considérable dans l'industrie pétrolière.

L'isomérisation du n-butane permet de produire de l'isobutane pour l'alkylation aliphatique des oléfines et la synthèse du MTBE (méthyl tertiobutyl éther), permettant respectivement de produire un alkylat d'indice d'octane élevé incorporable au pool essence et de fournir du MTBE utilisé comme dope d'octane.

L'isomérisation des paraffines normales $C_5$-$C_6$ permet de transformer ces paraffines de faible indice d'octane en isoparaffines d'indice d'octane élevé.

Trois types différents de catalyseurs sont traditionnellement utilisés pour réaliser cette réaction d'isomérisation :

- les catalyseurs de type Friedel et Crafts, tels que le chlorure d'aluminium, qui sont utilisés à des basses températures (environ 20 à 130°C) ;

- les catalyseurs à base de métal du groupe VIII sur alumine halogénée, qui sont utilisés à des températures moyennes (environ 150°C);

- les catalyseurs zéolithiques comprenant un métal du groupe VIII déposé sur une zéolithe, qui sont utilisés à des températures élevées (250°C et plus); ces catalyseurs conduisent à des gains d'octane moindres, mais présentent l'avantage d'être plus faciles de mise en oeuvre et plus résistants aux poisons ; néanmoins, de par leur plus faible acidité, ils ne peuvent pas être employés pour l'isomérisation du n-butane.

De nombreux brevets ont pour objet des catalyseurs monométalliques à base de platine déposé sur une alumine halogénée, ces catalyseurs étant utilisés dans des procédés d'isomérisation de paraffines normales dans lesquels les rapports molaires hydrogène/hydrocarbures ($H_2$/HC) sont relativement élevés afin de prévenir la désactivation par cokage liée aux réactions secondaires ou à la présence temporaire de soufre : par exemple, dans US-A-2 906 798, $H_2$/HC est supérieur à 0,17; dans US-A-2 993 398 ET US-A-3 791 960, $H_2$/HC est compris respectivement entre 0,2 et 10 et entre 0,1 et 15; dans US-A-4 113 789 et US-A-4 149 993, $H_2$/HC est égal à environ 0,2.

Plus récemment, il a été revendiqué dans US-A-4 804 803 la mise en oeuvre d'un catalyseur monométallique plus résistant à la désactivation, en particulier en présence de soufre, dans un procédé d'isomérisation de paraffines normales sans recyclage d'hydrogène, avec des rapports molaires $H_2$/HC dans l'effluent inférieurs à 0,05; ces faibles rapports minimisent le coût du procédé, puisqu'ils ne nécessitent pas de dispositif de séparation d'hydrogène et de compresseur pour le recycler.

Le brevet US-4 964 975 décrit un catalyseur comprenant 0,01 à 5% pds d'un métal du groupe IVA, 0,01 à 2% pds d'un métal noble et 0,1 à 10% pds d'un halogène, et un support, par exemple l'alumine éta. Ce catalyseur est utilisé en réformage catalytique.

L'objet de la présente invention est notamment l'utiisation d'un nouveau catalyseur bimétallique (comprenant au moins deux métaux) à base d'alumine chlorée présentant, outre une activité et une sélectivité importantes, une résistance au cokage et donc une stabilité considérablement accrues, permettant de le mettre en oeuvre dans un procédé d'isomérisation de paraffines normales $C_4$-$C_6$ ne nécessitant pas de recyclage d'hydrogène, avec une durée de cycle plus élevée.

Le catalyseur selon l'invention est à base d'alumine éta chlorée. comprenant 0,01 à 0,4% de préférence 0,015 à 0,025%, en poids d'un métal du groupe IV A de la classification périodique des éléments, tel que l'étain ou le germanium, de préférence l'étain, 0,05 à 0,6%, de préférence 0,15 à 0,4%, en poids d'un métal du groupe VIII de la classification périodique des éléments, de préférence le platine, et 1 à 12%, de préférence 4 à 10%, en poids de chlore.

L'alumine est l'alumine éta, qui conduit à un catalyseur régénérable du fait notamment de sa plus grande facilité de chloration liée à sa surface spécifique élevée d'environ 400m²/g (R.G.MC CLUNG, J.S. SOPKO et al 1990, NPRA, Annual Meeting San Antonio).

Le catalyseur selon l'invention est préparé par calcination de l'alumine éta, puis dépôt par échange anionique du métal du groupe VIII sur l'alumine éta calcinée, suivi du dépôt du métal du groupe IVA, le solide obtenu étant alors séché et soumis à chloration ainsi qu'à réduction.

Le métal du groupe VIII, de préférence le platine, est ainsi généralement déposé sur l'alumine calcinée par échange anionique avec une solution d'acide hexachloroplatinique,; d'autres composés que cet acide, comme le chloroplatinate d'ammonium, l'acide bromoplatinique ou le chlorure de platine peuvent également être utilisés.

Le métal du groupe IV A, en général l'étain ou le germanium, est alors déposé sur l'alumine déjà préimprégnée par le métal du groupe VIII, et calcinée et/ou réduite, en solution aqueuse ou en solution hydrocarbonée. Ce métal peut être introduit par l'intermédiaire de composés tels que les chlorures, les nitrates , les sulfates, les acétates, les complexes aminés d'étain ou de germanium en solution aqueuse et des alkyl ou aryl métaux d'étain ou de germanium tels que le tétrabutyl étain, le tétraméthyl étain, le diphényl étain, le tétrapropyl germanium le diphényl germanium en solution hydrocarbonée.

Une fois les métaux fixés sur l'alumine, le catalyseur subit avantageusement un traitement d'activation sous hydrogène à haute température, par exemple à une température comprise entre environ 300 et 500°C, afin d'obtenir une phase métallique active. La procédure de ce traitement sous hydrogène consiste par exemple en une montée lente de la température sous courant d'hydrogène jusqu'à la température maximale de réduction comprise généralement entre environ 300 et 500°C, de préférence entre environ 340 et 470°C, suivie d'un maintien de cette température pendant 1 à 6 heures, de préférence pendant 1,5 à 4,5 heures.

La chloration de l'alumine est effectuée en dehors de ou directement dans l'unité d'isomérisation par tout agent chlorant connu de l'homme du métier, tel que par exemple le tétrachlorure de carbone, le dichloroéthane, le dichloropropane, par exemple à une température comprise entre environ 200 et 350°C.

Le catalyseur selon l'invention est avantageusement mis en oeuvre dans un procédé d'isomérisation d'une charge riche en paraffines normales $C_4$ - $C_6$ sans recyclage d'hydrogène, ce qui constitue un autre objet de l'invention.

Le terme "riche" signifie que la charge contient au moins 50 %, de préférence au moins 80 %, de paraffines normales $C_4$ - $C_6$. Les charges préférées sont constituées essentiellement de paraffines normales $C_4$ - $C_6$. Ces charges sont par exemple du n-butane ou/et des coupes $C_5$ - $C_6$ par exemple issues de la distillation directe ou du réformage catalytique.

La charge est avantageusement séchée soigneusement et contient de préférence moins de 0,2 ppm d'eau et moins de 0,5 ppm de soufre et d'azote du fait de la sensibilité à l'eau et aux impuretés des catalyseurs au platine à base d'alumine chlorée. Pour atteindre de telles teneurs, on peut notamment utiliser des adsorbants spécifiques bien connus de l'homme du métier.

Dans le procédé d'isomérisation selon l'invention un mélange constitué de la charge et d'hydrogène est mis au contact dans une zone de réaction avec au moins un catalyseur conforme à l'invention et décrit précédemment dans des conditions d'isomérisation.

Les conditions opératoires d'isomérisation dans la zone de réaction, en particulier la température, dépendent notamment de la charge à traiter. Les isoparaffines étant favorisées à base température, d'un point de vue thermodynamique, le catalyseur sera d'autant plus performant qu'il travaillera à base température. La température dans la zone de réaction est habituellement comprise entre 40 et 230°C. Dans les cas où la charge contient essentiellement du n-butane ou essentiellement des paraffines normales $C_4$ - $C_6$, la température est habituellement comprise entre 130 et 230°C, de préférence entre 140 et 220°C. Dans les cas où la charge contient essentiellement des paraffines normales $C_5$ - $C_6$, l'isomérisation est plus facile à réaliser et la température est alors habituellement comprise entre 40 et 190°C, de préférence entre 70 et 170°C. La pression dans la zone de réaction peut varier dans un domaine assez large; elle est habituellement comprise entre 10 et 50 bars, de préférence entre 15 et 35 bars. Le débit de charge dans la zone de réaction peut également varier dans un domaine relativement large ; la vitesse spatiale est ainsi habituellement comprise entre 0,4 et 15 $h^{-1}$, de préférence entre 1 et 5 $h^{-1}$.

Une concentration en chlore dans la zone de réaction est maintenue entre 20 et 1000 ppm, de préférence entre 50 et 300 ppm ; en effet, afin de maintenir la teneur en chlore du catalyseur et donc son acidité, il est nécessaire d'injecter en continu dans la zone de réaction, par exemple par l'intermédiaire de la charge, un promoteur de chlore, tel que notamment le tétrachlorure de carbone, le chlorure d'hydrogène.

La quantité d'hydrogène mélangée à la charge est telle que l'effluent que l'on soutire de la zone de réaction possède un rapport molaire hydrogène/hydrocarbures ($H_2$/HC) inférieur à 0,05.

Le rapport molaire hydrogène/hydrocarbures ($H_2$/HC) de la charge est alors très faible, généralement inférieur à 0,1, ceci étant rendu possible notamment par la résistance au cokage améliorée du catalyseur utilisé.

L'effluent issu de la zone de réaction est séparé en un mélange d'hydrocarbures $C_4$ - $C_6$ comprenant les isoparaffines $C_4$ - $C_6$ obtenues et éventuellement les paraffines normales $C_4$ - $C_6$ non encore converties et un mélange gazeux contenant l'hydrogène (et des hydrocarbures légers), ledit mélange gazeux étant éliminé du procédé sans recyclage d'hydrogène. Le coût du procédé est très réduit car il ne nécessite pas de dispositif de séparation d'hydrogène et de compresseur pour le recycler. Les paraffines normales $C_4$ - $C_6$ non converties et contenues dans l'effluent issu de la zone de réaction sont généralement recyclées au moins en partie vers ladite zone de réaction.

La zone de réaction peut éventuellement comprendre au moins deux réacteurs en série dans lesquels les températures sont différentes, la température dans le second réacteur étant généralement inférieure à celle du premier réacteur.

Les exemples suivants illustrent l'invention sans toutefois en limiter la portée.

Les performances catalytiques sont exprimées par :

- les rapports

$$\frac{iC_x}{(i+n)C_x}$$

où x = 4,5 ou 6, $iC_x$ représente la quantité d'isoparaffines à x atomes de carbone dans l'effluent et $(i+n)C_x$ représente la quantité d'isoparaffines et de paraffines normales à x atomes de carbone dans l'effluent,

- et les approches à l'équilibre sur les différents isomères définies comme suit :

$$AEQi_x = \frac{\left(\dfrac{iC_x}{(i+n)C_x}\right) \text{ dans l'effluent}}{\left(\dfrac{iC_x}{(i+n)C_x}\right) \text{ à l'équilibre}}$$

avec $i_x$ = isoparaffine à x atomes de carbone (x = 4,5 ou 6).

EXEMPLE 1 : Catalyseur A (conforme à l'invetion)

Après calcination préalable de l'alumine éta, on dépose sur celle-ci 0,3 % de platine par échange anionique avec de l'acide hexachloroplatinique en présence d'HCl comme agent compétiteur; le solide ainsi préparé est calciné, puis réduit à 350°C. 200 ppm d'étain sont ensuite déposés en portant à reflux pendant 5 heures sous gaz inerte dans une solution d'heptane contenant du tetrabutyl étain le solide préparé précédemment. La solution est ensuite éliminée puis remplacée par une solution d'heptane fraiche. Le solide est lavé pendant 2 heures à reflux dans cette solution. La solution est ensuite éliminée, puis le solide est séché dans un évaporateur rotatif.

Le solide ainsi préparé est chargé dans un réacteur puis réduit directement à l'intérieur du réacteur à 450°C pendant 4 heures. On procède ensuite à la chloration, à une température de 280°C, par injection de $CCl_4$ à raison de 0,3 cm³ de $CCl_4$ par gramme de catalyseur. La teneur en chlore sur le catalyseur final est alors de l'ordre de 6 % poids.

EXEMPLE 2 : Catalyseur B (non conforme à l'invention).

Le catalyseur B diffère du catalyseur A préparé dans l'exemple 1 uniquement en ce qu'il ne contient pas d'étain. Les étapes de dépôt de platine, de traitements thermiques et de chloration sont identiques à celles décrites dans l'exemple 1.

EXEMPLE 3 : Test d'isomérisation de paraffines normales $C_5$ - $C_6$.

Les catalyseurs A et B préparés précédemment sont chacun testés en isomérisation d'une charge formée d'environ 60 % de paraffines normales $C_5$ et 40 % de paraffines normales $C_6$, ladite charge contenant 100 ppm de $CCl_4$ exprimés en poids de chlore pour maintenir la teneur en chlore du catalyseur utilisé.

Les conditions opératoires sont les suivantes :

| - Température | 150°C ; |
|---|---|
| - Pression | 20 bars ; |
| - v.v.h. | $2h^{-1}$ ; |
| - $H_2$/HC (dans l'effluent) | 0,05. |

Les performances obtenues après 24 et 2160 heures de fonctionnement sont reportées dans le tableau 1 : si le catalyseur A ne se désactive pratiquement pas, on constate au contraire une désactivation importante du catalyseur B.

EXEMPLE 4 : Test d'isomérisation de n-butane.

Les catalyseurs A et B préparés précédemment sont chacun testés en isomérisation d'une charge de n-butane, contenant 100 ppm de $CCl_4$ exprimés en poids de chlore.

Les conditions opératoires sont les suivantes :

| - Température | 200°C ; |
| - Pression | 20 bars ; |
| - v.v.h | $2h^{-1}$ ; |
| - $H_2$/HC (dans l'effluent) | 0,05. |

Les performances obtenues après 24 et 2160 heures de fonctionnement sont reportées dans le tableau 2 : si le catalyseur A ne se désactive pas, on constate au contraire une désactivation importante du catalyseur B.

EXEMPLE 4 :

Le catalyseur B préparé précédemment est testé dans les mêmes conditions que celles utilisées dans l'exemple 3, sauf que le rapport $H_2$/HC (dans l'effluent) est plus élevé et égal à 1.

Les performances obtenues après 24 et 2160 heures de fonctionnement sont reportées dans le tableau 3 : on ne constate pratiquement aucune désactivation du catalyseur B.

TABLEAU 1

| | Catalyseurs | | | |
| --- | --- | --- | --- | --- |
| | A | | B | |
| | Après 24 h. | Après 2160 h. | Après 24 h. | Après 2160 h. |
| $iC_5/(i + n)C_5$ | 0,762 | 0,760 | 0,762 | 0,70 |
| $iC_6/(i + n)C_6$ | 0,895 | 0,890 | 0,895 | 0,78 |
| AEQ $i_5$ (%) | 95,3 | 95,0 | 95,3 | 87,5 |
| AEQ 22 $DMC_4$ (%) | 87,8 | 87,0 | 87,8 | 75,0 |
| Craquage (% poids) (réaction secondaire) | 3,05 | 2,0 | 3,2 | 2,5 |
| (avec 22 $DMC_4$ = 2,2 diméthylbutane) | | | | |

TABLEAU 2

| | Catalyseurs | | | |
| --- | --- | --- | --- | --- |
| | A | | B | |
| | Après 24 h. | Après 2160 h. | Après 24 h. | Après 2160 h. |
| $iC_4/(i + n)C_4$ | 0,5 | 0,5 | 0,5 | 0,4 |
| AEQ $i_4$ (%) | 100 | 100 | 100 | 80 |
| Craquage (% poids) (réaction secondaire) | 2,2 | 2,0 | 2,5 | 2,2 |

TABLEAU 3

|  | Catalyseur B | |
|---|---|---|
|  | Après 24 h. | Après 2160 h. |
| $iC_5/(i + n)C_5$ | 0,762 | 0,760 |
| $iC_6/(i + n)C_6$ | 0,895 | 0,890 |
| AEQ $i_5$ (%) | 95,3 | 95,0 |
| AEQ 22 $DMC_4$ (%) | 87,8 | 87,0 |
| Craquage (% poids) (réaction secondaire) | 3,2 | 3,0 |

**Revendications**

1. Catalyseur à base d'alumine éta chlorée, comprenant 0,01 à 0,4 % en poids d'un métal du groupe IV A de la classification périodique des éléments, 0,05 à 0,6 % en poids d'un métal du groupe VIII de la classification périodique des éléments et 1 à 12 % en poids de chlore préparé par calcination de l'alumine éta, puis dépôt par échange anionique du métal du groupe VIII sur l'alumine éta calcinée, suivi du dépôt du métal du groupe IVA, le solide obtenu étant alors séché et soumis à chloration ainsi qu'à réduction.

2. Catalyseur selon la revendication 1, dans lequel la teneur pondérale en métal du groupe IV A est comprise entre 0,015 et 0,25 %, la teneur pondérale en métal du groupe VIII est comprise entre 0,15 et 0,4 % et la teneur pondérale en chlore est comprise entre 4 et 10 %.

3. Catalyseur selon l'une des revendications 1 à 2, dans lequel ledit métal du groupe VIII est le platine.

4. Catalyseur selon l'une des revendications 1 à 3, dans lequel ledit métal du groupe IV A est le germanium ou l'étain.

5. Catalyseur selon l'une des revendications 1 à 4, dans lequel ledit métal du groupe IV A est l'étain.

6. Catalyseur selon l'une des revendications 3 à 5, dans lequel le platine est déposé sur l'alumine éta au moyen d'une solution d'acide hexachloroplatinique en présence d'acide chlorhydrique.

7. Catalyseur selon l'une des revendications 1 à 6, dans lequel le solide obtenu après dépôt du métal du groupe VIII est calciné.

8. Catalyseur selon l'une des revendications 1 à 7, dans lequel le solide obtenu après dépôt du métal du groupe VIII est réduit.

9. Catalyseur selon l'une des revendications 1 à 8, dans lequel le métal du groupe IVA est introduit au moyen de composés choisis dans le groupe formé
par les chlorures, les nitrates, les sulfates, les acétates, les complexes aminés d'étain ou de germanium en solution aqueuse, les alkyls d'étain ou de germanium, les aryl d'étain ou de germanium.

10. Catalyseur selon l'une des revendications 1 à 9, dans lequel le métal du groupe IVA est introduit au moyen de composés choisis dans le groupe formé par le tétrabutyl étain, le tétraméthyl étain, le diphényl étain, le tétrapropyl germanium, le diphényl germanium, lesdits composés étant en solution hydrocarbonée.

11. Catalyseur selon l'une des revendications 1 à 10, dans lequel le solide obtenu après dépôts des métaux des groupes VIII et IVA est activé sous hydrogène.

12. Catalyseur selon l'une des revendications 1 à 11, dans lequel la chloration est effectuée à une température comprise entre 200 et 350°C par un agent chlorant choisi dans le groupe formé par le tétrachlorure de carbone, le dichloroéthane, le dichloropropane.

**13.** Procédé d'isomérisation d'une charge riche en paraffines normales $C_4$-$C_6$ sans recyclage d'hydrogène dans lequel :

- un mélange constitué de ladite charge et d'hydrogène est mis au contact dans une zone de réaction avec au moins un catalyseur selon l'une des revendications 1 à 12 dans des conditions d'isomérisation,

- une concentration en chlore dans ladite zone de réaction est maintenue entre 20 et 1000 ppm.

- la quantité d'hydrogène mélangé à ladite charge est telle que l'effluent issu de la zone de réaction possède un rapport molaire hydrogène/hydrocarbures inférieur à 0,05,

- l'effluent issu de la zone de réaction est séparé en un mélange d'hydrocarbures $C_4$-$C_6$ comprenant des isoparaffines $C_4$-$C_6$ et en un mélange gazeux contenant l'hydrogène, ledit mélange gazeux étant éliminé du procédé sans recyclage de l'hydrogène.

**14.** Procédé selon la revendication 13, dans lequel le rapport molaire hydrogène/hydrocarbures de la charge est inférieur à 0,1.

**15.** Procédé selon l'une des revendications 13 et 14, dans lequel dans la zone de réaction la température est comprise entre 40 et 230° C, la pression est comprise entre 10 et 50 bars et la vitesse spatiale est comprise entre 0,4 et 15 h$^{-1}$.

**16.** Procédé selon l'une des revendication 13 à 15, dans lequel les paraffines normales $C_4$-$C_6$ non converties et contenues dans l'effluent issu de la zone de réaction sont recyclées au moins en partie vers ladite zone de réaction.

**17.** Procédé selon l'une des revendications 13 à 16, dans lequel ladite zone de réaction comprend au moins deux réacteurs en série.

## Claims

**1.** Catalyst based on chlorinated eta alumina containing 0.01 to 0.4 % by weight of a metal of group IV A of the periodic classification of elements, 0.05 to 0.6 % by weiht of a metal of group VIII of the periodic classification of elements and 1 to 12 % by weight chlorine, prepared by calcination of eta alumina, then deposit of group VIII metal on calcined eta alumina by anion exchange, followed by deposit of group IV A metal, the obtained solid being then dried and submitted to chloration and reduction.

**2.** Catalyst according to claim 1, wherein the weight content of the metal of group IV A is between 0.015 and 0.25 %, the weight content of the metalof group VIII is between 0.15 and 0.4 % and the chlorine weight content is between 4 and 10 %.

**3.** Catalyst according to one of the claims 1 and 2, wherein the metal of group VIII is platinum.

**4.** Catalyst according to any one of the claims 1 to 3, wherein the metal of group IV A is germanium or tin.

**5.** Catalyst according to any on of the claims 1 to 4, wherein the metal of group IV A is tin.

**6.** Catalyst according to any one of the claims 3 to 5, wherein platinum is deposited on eta alumina with a solution of hexachloroplatinic acid in presence of chlorhydric acid.

**7.** Catalyst according to any one of the claims 1 to 6, wherein the solid obtained after deposit of group VIII metal is calcined.

**8.** Catalyst according to any one of the claims 1 to 7, wherein the solid obtained after deposit of group VIII metal is reduced.

**9.** Catalyst according to any one of the claims 1 to 8, wherein the group IV metal is introduced with compounds selected from the group formed by chlorides, nitrates, sulphates, acetates, amino complexes of tin or germanium in aqueous solution, tin or germanium alkyls, tin or germanium aryls.

EP 0 514 527 B1

**10.** Catalyst according to any one of the claims 1 to 9, wherein the group IV A metal is introduced with compounds selected from the group formed by tin tetrabutyl, tin tetramethyl, tin diphenyl, germanium tetrapropyl, germanium diphenyl, said compounds being in hydrocarbon solution.

**11.** Catalyst according to any one of the claims 1 to 10, wherein the solid obtained after deposits of G VIII and G IV A metals is activated under hydrogen.

**12.** Catalyst according to any one of the claims 1 to 11, wherein chlorination is carried out at a temperature between 200 and 350°C with a chlorinating agent selected from the group formed by carbon tetrachloride, dichloroethan, dichoropropan.

**13.** Process for the isomerization of a charge rich in normal C4-C6 paraffins without hydrogen recycling, wherein a mixture constituted by said charge and hydrogen is contacted in a reaction zone with at least one catalyst according to any one of the claims 1 to 12 under isomerization conditions, a chlorine concentation in said reaction zone is maintained at between 20 and 1000 ppm, the hydrogen quantity mixed with said charg is such that the effluent from the reaction zone has a hydrogen/hydrocarbon molar ration below 0.05, and the effluent from the reaction zone is separated into a $C_4$-$C_6$ hydrocarbon mixture containing $C_4$-$C_6$ isoparaffins and a gaseous mixture containing hydrogen, said gaseous mixture being eliminated from the process without hydrogen recycling.

**14.** Process according to claim 13, wherein the hydrogen/hydrocarbon molar ratio of the charge is below 0.1.

**15.** process according to either of the claims 13 and 14, wherein in the reaction zone the temperature is between 40 and 230°C, the pressure being between 10 and 50 bars and the space velocity between 0.4 and 15 $h^{-1}$.

**16.** Process according to any one of the claims 13 and 15, wherein the unconverted normal $C_4$-$C_6$ paraffins contained in the effluent from the reaction zone are at least partly recycled to said reaction zone.

**17.** Process according to any one of the claims 13 to 16, wherein said reaction zone has at least two reactors in series.

## Patentansprüche

**1.** Katalysator auf Basis von chloriertem Eta-Aluminiumoxid, umfassend 0,01 bis 0,4 Gew.% eines Metalls der Gruppe IV A des Periodensystems, 0,05 bis 0,6 Gew.% eines Metalls der Gruppe VIII des Periodensystems und 1 bis 12 Gew.% Chlor, hergestellt durch Kalzinierung von Eta-Aluminiumoxid, dann Abscheidung von Metall der Gruppe VIII durch anionischen Austausch auf dem kalzinierten Eta-Aluminiumoxid, gefolgt von der Abscheidung des Metalls der Gruppe IV A, wobei der erhaltene Feststoff dann getrocknet und einer Chlorierung sowie einer Reduktion unterworfen wird.

**2.** Katalysator nach Anspruch 1, worin der Gewichtsgehalt an Metall der Gruppe IV A zwischen einschließlich 0,015 und 0,25 % enthalten ist, der Gewichtsgehalt an Metall der Gruppe VIII zwischen einschließlich 0,15 und 0,4 % enthalten ist und der Gewichtsgehalt an Chlor zwischen 4 und 10 % enthalten ist.

**3.** Katalysator nach einem der Ansprüche 1 bis 2, worin das Metall der Gruppe VIII Platin ist.

**4.** Katalysator nach einem der Ansprüche 1 bis 3, worin das Metall der Gruppe IV A Germanium oder Zinn ist.

**5.** Katalysator gemäß einem der Ansprüche 1 bis 4, worin das Metall der Gruppe IV A Zinn ist.

**6.** Katalysator nach einem der Ansprüche 3 bis 5, worin das Platin auf dem Eta-Aluminiumoxid mittels einer Lösung von Hexachlorplatinsäure in Gegenwart von Chlorwasserstoffsäure abgeschieden wird.

**7.** Katalysator nach einem der Ansprüche 1 bis 6, worin der erhaltene Feststoff nach dem Abscheiden des Metalls der Gruppe VIII kalziniert wird.

**8.** Katalysator nach einem der Ansprüche 1 bis 7, worin der erhaltene Feststoff nach der Abscheidung des Metalls der Gruppe VIII reduziert wird.

**9.** Katalysator nach einem der Ansprüche 1 bis 8, worin das Metall der Gruppe IV A mittels Verbindungen eingeführt wird, die aus der durch die Chloride, die Nitrate, die Sulfate, die AcEtate, die aminierten Komplexe von Zinn oder

8

Germanium in wäßriger Lösung, die Zinn- oder Germaniumalkyle, die Zinn- oder Germaniumaryle gebildeten Gruppe ausgewählt sind.

10. Katalysator nach einem der Ansprüche 1 bis 9, worin das Metall der Gruppe IV A mittels von Verbindungen eingeführt wird, die aus der durch das Zinntetrabutyl, das Zinntetramethyl, das Zinndiphenyl, das Germaniumtetrapropyl, das Germaniumdiphenyl gebildeten Gruppe ausgewählt sind, wobei die Verbindungen in Kohlenwasserstofflösung vorliegen.

11. Katalysator nach einem der Ansprüche 1 bis 10, worin der erhaltene Feststoff nach den Abscheidungen der Metalle der Gruppe VIII und IV unter Wasserstoff aktiviert wird.

12. Katalysator nach einem der Ansprüche 1 bis 11, worin die Chlorierung bei einer Temperatur bewirkt wird, die zwischen 200 und 350°C liegt, durch ein Chlorierungsmittel, das aus der durch das Tetrachlorkohlenstoff, das Dichlorethan, das Dichlorpropan gebildeten Gruppe ausgewählt ist.

13. Verfahren zur Isomerisierung einer an $C_4$-$C_6$-n-Paraffinreichen Charge ohne Recyclierung von Wasserstoff, worin:

- eine aus der Charge und Wasserstoff gebildete Mischung in einer Reaktionszone mit wenigstens einem Katalysator nach einem der Ansprüche 1 bis 12 unter Isomerisierungsbedingungen in Kontakt gebracht wird,

- eine Konzentration an Chlor in der Reaktionszone zwischen 20 und 1000 ppm aufrechterhalten wird;

- die mit der Charge vermischte Wasserstoffmenge derart ist, daß der die Reaktionszone verlassende Abstrom ein Molverhältnis von Wasserstoff/Kohlenwasserstoff von unter 0,05 besitzt;

- der die Reaktionszone verlassende Abstrom in eine $C_4$-$C_6$-Kohlenwasserstoffmischung, welche $C_4$-$C_6$-Isoparaffine umfaßt, und in eine gasförmige Mischung, welche den Wasserstoff enthält, aufgetrennt wird, wobei die gasförmige Mischung aus dem Verfahren ohne Recyclierung des Wasserstoffes entfernt wird.

14. Verfahren nach Anspruch 13, worin das Molverhältnis von Wasserstoff/Kohlenwasserstoffe der Charge unter 0,1 liegt.

15. Verfahren nach einem der Ansprüche 13 und 14, worin in der Reaktionszone die Temperatur zwischen einschließlich 14 und 230°C enthalten ist, der Druck zwischen einschließlich 10 und 50 bar enthalten ist und die Raumgeschwindigkeit zwischen einschließlich 0,4 und 15 $h^{-1}$ enthalten ist.

16. Verfahren nach einem der Ansprüche 13 bis 15, worin die nicht umgewandelten und in dem die Reaktionszone verlassenden Abstrom enthaltenen $C_4$-$C_6$-n-Paraffine wenigstens teilweise zur Reaktionszone zurückgeführt werden.

17. Verfahren nach einem der Ansprüche 13 bis 16, worin die Reaktionszone wenigstens zwei Reaktoren in Reihe umfaßt.